# EUROPEAN PATENT APPLICATION

(11) **EP 4 074 205 A1**
(43) Date of publication of application: **19.10.2022**
(21) Application number: 20897942.7
(22) Date of filing: 23.01.2020
(51) Int. Cl.: A41C 3/10

(54) **UNDERGARMENT**

(30) Priority: 13.12.2019 JP 2019225572
(71) Applicant: Maeda, Makoto, Otsu-shi, Shiga, 520-0023 (JP)
(72) Inventor: Maeda, Makoto, Otsu-shi, Shiga, 520-0023 (JP)
(74) Representative: Plasseraud IP
(86) International application number: PCT/JP2020/002359
(87) International publication number: WO 2021/117259

(57) **Abstract**

It is an object of the present invention to provide an underwear capable of preventing a chest of a wearer from jiggling.

The underwear (100) according to the present invention comprises a pair of cup portions (110), a wire (170), and a wire length adjusting portion (160). The pair of cup portions protrude to a same side. The wire length adjusting portion is disposed between the foot of the pair of cup portions. Then, the wire extends from the opposite side of the wire length adjusting portion side of the cup portion to the wire length adjusting portion. The wire length adjusting portion can adjust a length L of the wire from the opposite side of the wire length adjusting portion side of the pair of cup portions to the wire length adjusting portion.

## Description

### [TECHNICAL FIELD]

The present invention relates to an underwear.

### [BACKGROUND ART]

"A brassiere comprising: a pair of cup portions; a connecting portion connecting a front center side of the cup portion; a wing portion; and a shoulder strap portion whose end is connected to the pair of cup portions and the wing portion; wherein the cup portion has at least a pad, the pad has a pad high stress portion, the pad high stress portion extends along an upper edge of the pad, a lower edge of the pad, or both, and a stretching stress S2 of the pad high stress portion/a stretching stress S1 of a pad bust top portion of the pad is 2/1 to 80/1." has been proposed in the past (for example, see Japanese Unexamined Patent Publication No. 2014-163018, etc.).

### [PRIOR ART DOCUMENT]

[PATENT DOCUMENT 1] Japanese Unexamined Patent Publication No. 2014-163018

### [SUMMARY OF THE INVENTION]

### [PROBLEM TO BE SOLVED BY THE INVENTION]

Incidentally, even when a wearer wears a brassiere during exercise or the like, a chest of the wearer often jiggles. When the wearer's chest jiggles, a movement during exercise and the like is hindered, the Cooper's ligament may be damaged and the chest of wearer may droop. According to the specification of the high stress portion of the upper and lower edges of the pad as described in Patent Document 1, it is difficult to say that the jiggling of the chest of the wearer can be sufficiently prevented.

It is an object of the present invention to provide an underwear capable of preventing a chest of a wearer from jiggling.

### [MEANS FOR SOLVING THE PROBLEMS]

An underwear according to a first aspect of the present invention comprises a pair of cup portions, a wire, and a wire length adjusting portion. The pair of cup portions protrude to a same side. The wire length adjusting portion is disposed between the foot of the pair of cup portions. Then, the wire extends from the opposite side of the wire length adjusting portion side of the cup portion to the wire length adjusting portion. The wire length adjusting portion can adjust a length of the wire from the opposite side of the wire length adjusting portion side of the pair of cup portions to the wire length adjusting portion. Incidentally the "underwear" as used herein refers to clothing having the cup portion (e.g., brassieres, sportswear such as tank tops, swimsuits, body suits, camisoles, etc.).

According to the above configuration, the chest of the wearer can be held down by the wire length adjusting portion and the wire. Therefore, in this underwear, it is possible to prevent the chest of the wearer from jiggling.

The underwear according to a second aspect of the present invention is the underwear according to the first aspect, and in a front view, the wire passes through a position lower than a height position of a vertex of the cup portion and passes through a position higher than a height position of a lower end portion of the cup portion. Incidentally, in a front view, the wire preferably passes through a point within a range of 1cm or more and 2cm or less from the vertex of the cup portion in a direction directly downward from the vertex of the cup portion, and more preferably passes through a point within a range of 1.5cm or more and 2cm or less.

According to the above configuration, the lower side of the chest of the wearer can be held down. Therefore, in this underwear, it is possible to prevent the chest of the wearer from drooping and to prevent the chest of the wearer from jiggling.

The underwear according to a third aspect of the present invention is the underwear according to the first aspect or the second aspect, and the wire passes through an inside of the cup portion.

According to the above configuration, since the wire can be hidden in the cup portion, it is possible to prevent the appearance of the underwear from being impaired. In addition, according to the above configuration, it is possible to prevent the wire from directly touching the skin of the wearer. Therefore, in this underwear, it is possible to prevent the wearer from feeling discomfort.

The underwear according to the fourth aspect of the present invention is the underwear according to any one of the first to third aspects, and the wire length adjusting portion includes a communication unit and a driving unit. The communication unit receives an instruction from an external terminal. The driving unit adjusts the length of the wire from the opposite side of the wire length adjusting portion side of the pair of cup portions to the wire length adjusting portion in response to the instruction from the external terminal.

According to the above configuration, the external terminal (e.g., a smart phone terminal, a tablet terminal, a wearable terminal, a personal computer or the like) can be operated to drive the driving unit (e.g., a small motor or the like). Therefore, the wearer can adjust the length of the wire from the opposite side of the wire length adjusting portion side of the pair of cup portions to the wire length adjusting portion without touching the wire length adjusting portion.

The underwear according to a fifth aspect of the present invention is the underwear according to the fourth aspect, and the wire length adjusting portion further includes a heart rate measuring unit. The heart rate measuring unit measures a heart rate. The communication unit transmits the heart rate measured by the heart rate measuring unit to the external terminal.

According to the above configuration, the heart rate of the wearer can be measured by the heart rate measuring unit (e.g., an electrocardiographic type that detects an electrocardiogram by installing electrodes, an optical type that detects blood flow changes, or the like). Therefore, the wearer can perform motion or the like while conscious of the heart rate.

### [BRIEF DESCRIPTION OF THE DRAWINGS]

[Fig. 1] It is a front view of a brassiere according to an embodiment of the present invention.
[Fig. 2] It is a plan view of the brassiere according to an embodiment of the present invention. It should be noted that the drawing is simplified by omitting some members such as a shoulder strap portion and the like.
[Fig. 3] It is a front view of a brassiere according to a modification.
[Fig. 4] It is a schematic configuration diagram of a wire adjusting portion and an external terminal according to an embodiment of the present invention.

### [DESCRIPTION OF THE PREFERRED EMBODIMENTS]

### <Structure of a brassiere according to an embodiment of the present invention>

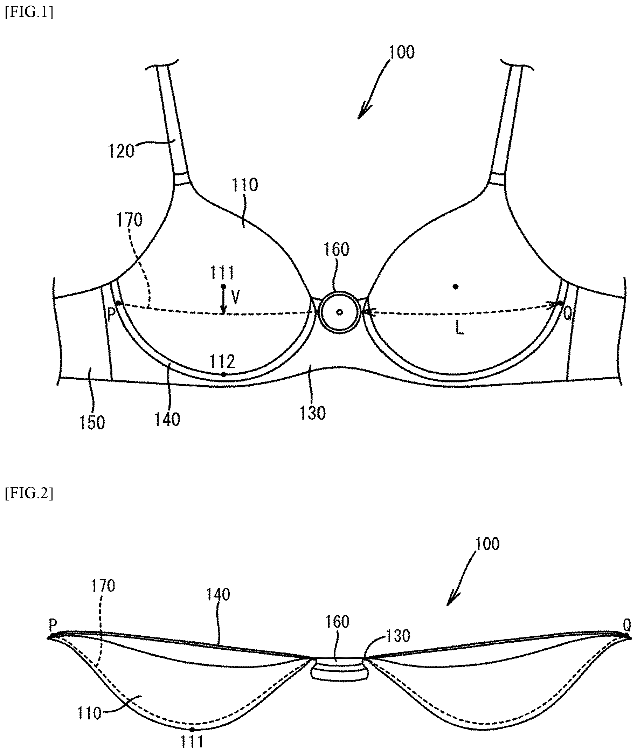

As shown in FIG. 1, the brassiere 100 according to an embodiment of the present invention is mainly composed of a cup portion 110, a shoulder strap portion 120, a base portion 130, a cup support portion 140, a belt portion 150, a wire length adjusting portion 160, a wire 170, and the like. Hereinafter, these components will be described in detail.

### (1) Cup portion

The cup portion 110 is a bell-shaped portion covering the chest of the wearer, and is provided in a pair of left and right so as to protrude to the same side as shown in FIGS. 1 and 2. The cup portion 110 is mainly composed of a cup front cloth and a cup back cloth, and the wire 170 (to be described later) passes through between the cup front cloth and the cup back cloth. Incidentally, as shown in FIGS. 1 and 2, the cup portion 110 has a vertex 111 of a bulge.

### (2) Shoulder strap portion

The shoulder strap portion 120 is a portion connected to the cup portion 110 and the belt portion 150 (to be described later), and is provided in a pair of right and left as shown in FIG. 1. The shoulder strap portion 120 is formed of a material having elasticity. Incidentally, in order to adjust the length of the shoulder strap portion 120 from the cup portion 110 to the belt portion 150, a length adjusting tool such as an adjuster may be provided on the shoulder strap portion 120.

### (3) Base portion

The base portion 130 is a portion for connecting the pair of cup portions 110, and is connected to the cup support portion 140 (to be described later) as shown in FIGS. 1 and 2.

### (4) Cup support portion

The cup support portion 140 is a portion for supporting the cup portion 110, and is a portion provided along the lower edge portion of the cup portion 110 as shown in FIGS. 1 and 2. Incidentally, a shape retaining wire which is formed of metal, resin, or the like may be accommodated in the cup support portion 140.

### (5) Belt portion

The belt portion 150 is a belt-shaped portion covering the back of the wearer, and is connected to the lateral outside of the base portion 130 as shown in FIG. 1. Incidentally, the belt portion 150 is formed in a series without being locked by a locking tool such as a hook at the back portion of the wearer. The belt portion 150 is formed of a material having elasticity.

### (6) Wire length adjusting portion

The wire length adjusting portion 160 is a portion for adjusting the length L of the wire 170, and is provided between the foot of the pair of cup portions 110 and on the front side of the base portion 130 as shown in FIGS. 1 and 2. Incidentally, the length L of the wire 170 is not the length of the wire 170 itself, but is the length of the wire 170 from the point P (Q) existing on the opposite side of the wire length adjusting portion 160 side of the cup portion 110 to the wire length adjusting portion 160. Incidentally, in the brassiere 100 according to the present embodiment, the points P and Q are located at the outer portion of the cup support portion 140 as shown in FIGS. 1 and 2. Further, the wire length adjusting portion 160, for example, is composed of a reel or a rotatable dial or the like. According to such a configuration, it is possible to shorten the length L of the wire 170 by rotating the dial and winding the wire 170 to the reel in a state where the dial is pressed against the skin side of the wearer, and it is possible to return the length L of the wire 170 to the length of the initial state by pulling the dial to the front side. Here, the initial state refers to a state in which the length L of the wire 170 is not shortened by the wire length adjusting portion 160. As such the wire length adjusting portion 160, a commercially available one (for example, Boa (registered trademark) manufactured and sold by Bore Technology Corporation, TGF (registered trademark) manufactured and sold by Japana Corporation, or the like) can be appropriately selected and used.

### (7) Wire

The wire 170 is formed of a metal, a resin, or the like. Incidentally, as shown in FIGS. 1 and 2, one wire 170 extends from point P (Q) to point Q (P) via the wire length adjusting portion 160. Here, the ends of the wires 170 are fixed so as not to move at points P and Q, respectively. As shown in FIGS. 1 and 2, in the front view, the wire 170 passes through a position lower than the height position of the vertex 111 of the cup portion 110 and passes through a position higher than the height position of the lower end 112 of the cup portion 110. Incidentally, at this time, in the front view, it is preferable that the wire 170 passes through a point within a range of 1cm or more and 2cm or less from the vertex 111 of the cup portion 110 in the direction V directly downward from the vertex 111 of the cup portion 110, and more preferably passes through a point within a range of 1.5cm or more and 2cm or less. The wire 170 passes through the inside of the cup portion 110 (between the cup front cloth and the cup back cloth), and is along to the curvature of the cup portion 110 in the initial state. Further, the diameter of the wire 170 is preferably within a range of 0.5mm or more and 2mm or less, and more preferably within a range of 1mm or more and 2mm or less.

As described above, by configuring the wire length adjusting portion 160 and the wire 170, when the length L of the wire 170 is shortened by the wire length adjusting portion 160, the wire 170 can collapse the bulge of the cup back cloth of the cup portion 110 and hold down the chest of the wearer.

### <Features of the brassiere according to the embodiment of the present invention>

(1) In the brassiere 100 according to the embodiment of the present invention, one wire 170 extends from a point P(Q) to a point Q(P) via the wire length adjusting portion 160. Then, the wire length adjusting portion 160 is disposed between the foot of the pair of cup portions 110, and can adjust the length L of the wire 170. Therefore, in this brassiere 100, the chest of the wearer can be held down. Therefore, in this brassiere 100, it is possible to prevent the jiggling of the chest of the wearer.
(2) In the brassiere 100 according to the embodiment of the present invention, the wire 170 passes through a point within a range of 1cm or more and 2cm or less from the vertex 111 of the cup portion 110 in the direction V directly downward from the vertex 111 of the cup portion 110 when viewed from the front. Therefore, in this brassiere 100, the lower side of the chest of the wearer can be held down. Therefore, in this brassiere 100, it is possible to prevent the chest of the wearer from drooping and to prevent the chest of the wearer from jiggling.
(3) In the brassiere 100 according to the embodiment of the present invention, since the wire 170 can be hidden in the cup portion 110 (between the cup front cloth and the cup back cloth), it is possible to prevent the appearance of the brassiere 100 from being impaired. In addition, in the brassiere 100, it is possible to prevent the wire 170 from directly touching the skin of the wearer. Therefore, in this brassiere 100, it is possible to prevent the wearer from feeling discomfort.

### <Modifications>

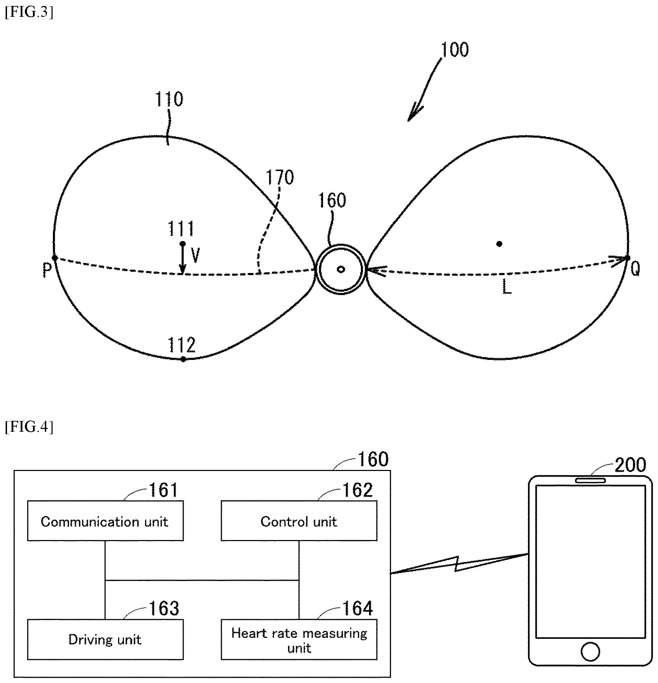

(A) In the brassiere 100 according to the above embodiment, the wire 170 passes through the inside of cup portion 110. However, the wire 170 may pass through the skin side of the cup portion 110 or the front side of the cup portion 110. Incidentally, when the wire 170 passes through the skin side of the cup portion 110, a wire loop or the like for passing the wire 170 through the skin side of the cup 110 is preferably provided inside the cup portion 110. However, such the wire loop or the like may not be provided.
(B) In brassiere 100 according to the above embodiment, one wire 170 extends from point P (Q) to point Q (P) via the wire length adjusting portion 160. However, one wire extends from the point P to the wire length adjusting portion 160, and the other wire may extend from the point Q to the wire length adjusting portion 160.
(C) In the brassiere 100 according to the above embodiment, a part of the base portion 130 is provided between the foot of the pair of cup portions 110. However, as shown in FIG. 3, the base portion 130 may not be provided. In this case, the pair of cup portions 110 is connected by the wire 170 alone, or the pair of cup portions 110 is connected by bonding the cup portion 110 and the wire length adjusting portion 160, etc.
(D) In the brassiere 100 according to the above embodiment, the shoulder strap portion 120 is connected to the cup portion 110 and the belt portion 150. Further, the cup support portion 140 is provided along the lower side portion of the cup portion 110. Further, the belt portion 150 is connected to the lateral outside of the base portion 130. However, as shown in FIG. 3, the shoulder strap portion 120, the cup support portion 140, or the belt portion 150 may not be provided. Incidentally, in the case where the shoulder strap portion 120 and the belt portion 150 are not provided, it is preferable that the cup portion 110 is formed from a material (e.g., silicon or the like) having stickiness and elasticity so as not to cause the brassiere 100 to fall from the chest of the wearer.
(E) In the brassiere 100 according to the above embodiment, the belt portion 150 is formed in a series without being locked by a locking tool such as a hook at the back of the wearer. However, the belt portion extending from the lateral outside of the base portion 130 may be locked with each other by a locking tool such as a hook, and the tightening condition during wearing may be finely adjusted.
(F) In the brassiere 100 according to the above embodiment, the wire length adjusting portion 160, for example, is composed of a reel or a rotatable dial or the like, and the length L of the wire 170 is shortened by rotating the dial and winding the wire 170 to the reel. However, the wire length adjusting portion 160 is not limited to a configuration of a reel or a dial or the like, and may be a configuration that can shorten the length L of the wire 170.
(G) While the present invention has been applied to the brassiere 100 in the above embodiment, the present invention may be applied to clothing having a cup portion (e.g., sportswear such as tank tops, swimsuits, body suits, camisoles, or the like)
(H) As shown in FIG. 4, the wire length adjusting portion 160 according to the above embodiment may further include a communication unit 161, a control unit 162, a driving unit 163, a heart rate measuring unit 164, and the like. As shown in FIG. 4, the wire length adjusting portion 160 and an external terminal 200 may communicate with each other. Here, the communication unit 161 is a communication interface for performing wireless communication with the external terminal 200. The communication unit 161 transmits the data (e.g., the heart rate of the wearer, etc.) from the control unit 162 to the external terminal 200, and receives the data (e.g., an instruction to the wire length adjusting portion 160, etc.) from the external terminal 200 and transfers the data to the control unit 162. The control unit 162 is a central processing unit (CPU) and controls each unit of the wire length adjusting portion 160. The driving unit is, for example, a small motor or the like. The driving unit is driven under control by the control unit 162 which receives the instruction from the external terminal 200, and adjust the length L of the wire 170. The heart rate measuring unit is, for example, an electrocardiographic type sensor that detects electrocardiogram by installing electrodes, an optical sensor that detects blood flow changes, or the like, and measures the heart rate of the wearer. Incidentally, although not shown in FIG. 4, the wire length adjusting portion 160 further includes a storage unit. The storage unit is various RAM (Random Access Memory) and various ROM (Read Only Memory) or the like, and stores programs to be executed by the control unit 162 and various data. The external terminal 200 is, for example, a smart phone terminal, a tablet terminal, a wearable terminal, a personal computer, or the like. The external terminal 200 transmits the instruction or the like to the wire length adjusting portion 160, receives the heart rate measured by the heart rate measuring unit 164 and displays the heart rate on a display or the like.

Here, a case of adjusting the length L of the wire 170 by operating the external terminal 200 and driving the driving unit 163 will be described. First, the control unit 162 receives the instruction for adjusting the length L of the wire 170 from the external terminal 200 via the communication unit 161. Next, the control unit 162 drives the driving unit 163 in accordance with the received instruction. Then, by the driving unit 163 is driven, it is possible to adjust the length L of the wire 170. In this manner, when this modification is applied, the wearer can adjust the length L of the wire 170 without touching the wire length adjusting portion 160. That is, the brassiere according to this modification is convenient because the length L of the wire 170 can be adjust even after wearing clothes on the brassiere.

Here, a case of measuring the heart rate of the wearer by the heart rate measuring unit 164 will be described. First, the control unit 162 causes the heart rate measuring unit 164 to measure the heart rate of the wearer. Next, the control unit 162 transmits the measured heart rate to the external terminal 200 via the communication unit 161. Then, the external terminal 200 displays the received heart rate on a display or the like. Incidentally, the heart rate measuring unit 164 may always measure the heart rate of the wearer, or may start measuring the heart rate of the wearer after the instruction from the external terminal 200 is received. In addition, the heart rate measuring unit 164 may not be configured in the wire length adjusting portion 160, but may be independently configured in the brassiere 100 according to the above embodiment (in this case, the independent heart rate measuring unit 164 includes a communication unit or the like for wireless communication with the external terminal 200). In this manner, when this modification is applied, the wearer can perform exercise or the like while conscious of the heart rate.

(I) Although not specifically mentioned in the above embodiment, a configuration for realizing a music playback function, a call function, a speed and distance measurement function in running, cycling, or the like, a function of an electronic payment system, or the like may be incorporated in the wire length adjusting portion 160 of the brassiere 100 according to the above embodiment. In this case, it is preferable to configure the communication unit 161, the control unit 162, the storage unit, the external terminal 200, and the like as described in Modification (H). As for the music playback function, the music stored in the storage unit can be played back, for example, by using a bone conduction speaker. The call function can be realized by using, for example, a bone conduction speaker or a microphone. The speed and distance measurement functions in running, cycling, or the like can be realized by using, for example, GPS (Global Positioning System). With this measurement function, it is possible to measure the speed and distance from the route or the like that the wearer of the brassiere 100 has passed. The result of the measured speed and distance is preferably displayed on the external terminal 200. The electronic payment system can be realized, for example, by performing communication by the communication unit 161 or the like.

### <Note>

The above Modifications (A) to (I) may be applied alone or in combination.

### [REFERENCE SIGNS LIST]

- 100: Brassiere (Underwear)
- 110: Cup portion
- 111: Vertex
- 112: Lower end
- 120: Shoulder strap portion
- 130: Base portion
- 140: Cup support portion
- 150: Belt portion
- 160: Wire length adjusting portion
- 161: Communication unit
- 162: Control unit
- 163: Driving unit
- 164: Heart rate measuring unit
- 170: Wire
- 200: External terminal
- L: Length
- P,Q: Point
- V: Direction

## Claims

1. An underwear comprising:
a pair of cup portions protruding to a same side;
a wire; and
a wire length adjusting portion disposed between the foot of the pair of cup portions;
wherein the wire extends from the opposite side of the wire length adjusting portion side of the cup portion to the wire length adjusting portion, and
the wire length adjusting portion can adjust a length of the wire from the opposite side of the wire length adjusting portion side of the pair of cup portions to the wire length adjusting portion.

2. The underwear according to claim 1, wherein, in a front view, the wire passes through a position lower than a height position of a vertex of the cup portion and passes through a position higher than a height position of a lower end portion of the cup portion.

3. The underwear according to claim 1 or 2, wherein, in a front view, the wire passes through a point within a range of 1cm or more and 2cm or less from the vertex of the cup portion in a direction directly downward from the vertex of the cup portion.

4. The underwear according to any one of claims 1 to 3, wherein the wire passes through an inside of the cup portion.

5. The underwear according to any one of claims 1 to 4, wherein the wire length adjusting portion includes a communication unit for receiving an instruction from an external terminal and a driving unit for adjusting the length in response to the instruction from the external terminal.

6. The underwear according to claim 5, wherein
the wire length adjusting portion further includes a heart rate measuring unit for measuring a heart rate, and
the communication unit transmits the heart rate measured by the heart rate measuring unit to the external terminal.
